# EUROPÄISCHE PATENTSCHRIFT

(11) **EP 4 161 462 B1**
(45) Veröffentlichungstag und Bekanntmachung des Hinweises auf die Patenterteilung: **11.03.2026**
(21) Anmeldenummer: 21732847.5
(22) Anmeldetag: 09.06.2021
(51) Int. Cl.: A61F 11/00, A61H 7/00

(54) **VORRICHTUNG ZUR BEHANDLUNG EINES TINNITUS-LEIDENS**
DEVICE FOR TREATING A TINNITUS AFFLICTION
DISPOSITIF DE TRAITEMENT D'ACOUPHÈNES

(30) Priorität: 09.06.2020 AT 505022020
(43) Veröffentlichungstag der Anmeldung: 12.04.2023
(73) Patentinhaber: Grübl, Klaus, 5280 Braunau/Inn (AT)
(72) Erfinder: Grübl, Klaus, 5280 Braunau/Inn (AT)
(74) Vertreter: Gassner, Birgitta
(86) Internationale Anmeldenummer: PCT/EP2021/065486
(87) Internationale Veröffentlichungsnummer: WO 2021/250104

(56) Entgegenhaltungen:
- WO-A1-2019/158674
- DE-A1- 10 139 865
- JP-A- 2009 095 627
- JP-A- 2020 039 663

## Beschreibung

### Technisches Gebiet

Die Erfindung betrifft eine Vorrichtung zur Behandlung eines Tinnitus-Leidens und/oder Reduzierung von Stress.

### Stand der Technik

Der Tinnitus ist eine auditive Wahrnehmung, die zusätzlich zu dem auf das Ohr einwirkenden Schall ein- oder beidseitig wahrgenommen wird. Diese Wahrnehmung beruht auf einer Störung der Hörfunktion. Die Art der scheinbaren Geräusche ist sehr vielfältig: Die auditiven Eindrücke werden als Brummton oder Pfeifton, Zischen, Rauschen, Knacken oder Klopfen beschrieben.

Tinnitus beeinträchtigt die Lebensqualität. Bisherige Behandlungsmethoden (dazu gehören verschiedene Formen der akustischen Stimulation, verhaltenstherapeutische Ansätze, kombinierte Therapieansätze, die akustische Stimulation und verhaltenstherapeutische Elemente beinhalten (zum Beispiel die Tinnitus-Retraining-Therapie), medikamentöse Therapieverfahren, Physiotherapie, magnetische und elektrische Gehirnstimulationsverfahren) greifen nicht immer, oder nicht gut genug. Für die meisten der angebotenen Therapien liegt kein Wirknachweis durch ausreichend große, Placebo kontrollierte Studien vor.

Im Stand der Technik ist beispielsweise eine Ohrvorrichtung beschrieben, die schnelle Druckänderungen aufgrund von schnellen Höhenänderungen in Flugzeugen ausgleichen soll um Ohrschmerzen zu vermeiden. GB2450931 beschreibt beispielsweise Ohrenschützer, in denen durch Verwendung einer Luftpumpe der Druck reguliert werden kann.

Bei Gehörschützern, die als Schallschutz getragen werden, tritt nach längerem Gebrauch ein Wärmestau auf, der zu einer übermäßigen Transpiration führen kann. Um einen Luftaustausch zu ermöglichen, ist eine Belüftungsöffnung auf der Kapsel des Gehörschutzes vorgesehen. Durch Veränderung des Volumens des Kapselinnenraums wird so der Luftaustausch ermöglicht (DE2910315).

In CN205598091 (U) wird eine Vorrichtung, umfassend eine Brille, ein Bluetooth-Headset und Kopfhörer, beschrieben. Über diese Vorrichtung kann beispielsweise mittels einer Software Umgebungsklänge, Töne oder Musik zur Behandlung von Tinnitus eingespielt werden.

WO2015164889 beschreibt einen Kopfhörer der im Gebrauch innerhalb oder nahe eines Ohrkanals eines Benutzers angeordnet werden kann und eine Ohrschleife um den Ohrhörer am Ohr des Benutzers zu befestigen. Die Ohrschleife umfasst ein eine aufblasbare Blase um den Ohrhörer im Gebrauch besser am Ohr des Benutzers zu befestigen. US20170303031 beschreibt Kopfhörer umfassend ein aufblasbares Montagesystem um das Gehäuse des Kopfhörers besser am menschlichen Ohr zu befestigen. CN206743497 beschreibt einen Kopfhörer mit einem aufblasbaren Schwellkörper, der jedoch im Innenohr angebracht wird.

WO2019158674 beschreibt eine Vorrichtung zur Behandlung eines Tinnitus-Leidens, umfassend einen Körper, der so bemessen ist, dass er an oder im nahen Umfeld der Ohrmuschel befestigt werden kann, dadurch gekennzeichnet, dass dieser Körper mindestens einen punktuellen Schwellkörper aufweist, wodurch die Stellung der Ohrmuschel verändert wird. Bevorzugterweise wird der punktuelle Schwellkörper auf einem Brillenbügel angebracht. Diese Vorrichtung hat jedoch den Nachteil, dass sie nur sehr ungenau und schwierig an die Bedürfnisse der jeweiligen Träger angepasst werden kann.

Tinnitus kann durchaus heilbar sein. Insbesondere bei akutem Tinnitus sind die Aussichten auf Heilung gut. Allerdings gibt es keine genauen Zahlen darüber, wie viele Tinnitusbetroffene in welcher Form geheilt werden. Als geheilt kann sich ein Tinnituspatient betrachten, wenn sein Ohrgeräusch verschwunden ist. Es besteht daher nach wie vor der Bedarf nach einer zielgerichteten, dauerhaften und erfolgreichen Behandlung von Tinnitus. Aufgabe der vorliegenden Erfindung ist es daher, eine Vorrichtung und deren Verwendung bereitzustellen, in der der Druckpunktkörper einfach zu positionieren ist, vom Benutzer an seine individuellen Bedürfnisse angepasst werden kann und angenehm zu tragen ist.

### Kurze Zusammenfassung der Erfindung

Die Aufgabe wird durch die unabhängigen Ansprüche gelöst. Die vorliegende Erfindung umfasst somit eine neue Vorrichtung zur Behandlung von Tinnitus und/oder Reduzierung von Stress, wobei diese Vorrichtung die Stellung der Ohrmuschel verändert und so der Schalleintrittswinkel geändert wird. Durch diese Änderung am Außenohr wird das Tinnitus-Leiden gemildert und/oder ganz geheilt.

Insbesondere betrifft die vorliegende Erfindung eine Vorrichtung zur Behandlung eines Tinnitus-Leidens und/oder Reduzierung von Stress umfassend einen Ohrbügel bestehend aus einem oberen 1 und unteren Bügel 2, und einem Druckpunktkörperkörper 3. Die Vorrichtung ist so bemessen ist, dass sie hinter der Ohrmuschel (Außenohr) getragen werden kann. Der Druckpunktkörper 3 kann in unterschiedlichen Größen und Formen gefertigt sein. Die Position des Druckpunktkörpers kann auf dem Bügel individuell auf den jeweiligen Träger eingestellt werden. Eventuell notwendige Nachjustierungen der Position des Druckpunktkörpers können vom Träger selbständig vorgenommen werden.

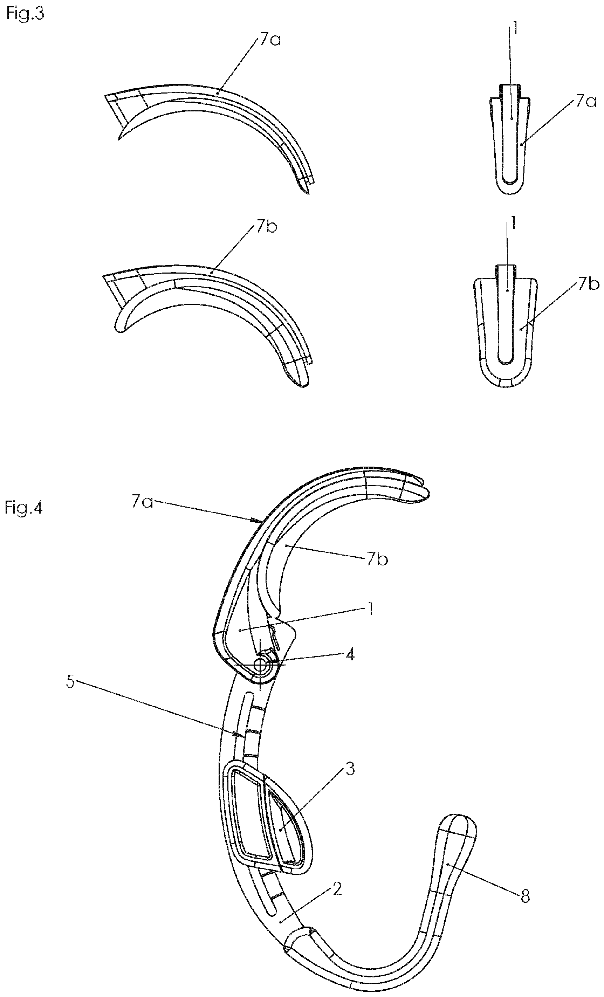

Eine Ausführungsform umfasst die Vorrichtung wie hierin beschrieben, wobei die Vorrichtung aus einem oberen und einem unteren Bügel besteht, die mittels einer Steckverbindung 4 verbunden sind. Diese Verbindung ermöglicht, dass die Stellung des oberen und unteren Bügels zueinander verändert werden kann. So wird gewährleistet, dass die Vorrichtung geöffnet werden kann (Fig. 4) um ein einfacheres Anbringen hinter dem Ohr zu ermöglichen.

Durch diese bewegliche Ausführungsform kann erreicht werden, dass die Vorrichtung an dem Ohr des Benutzers in der Stellung gehalten werden kann, in welcher der Druckpunktkörper an der gewünschten Position hinter dem Außenohrs des Benutzers positioniert werden kann. Die Vorrichtung kann in dieser Stellung gehalten werden, ohne dass permanent eine Klemmkraft oder dergleichen auf das Ohr des Benutzers wirken muss. Dadurch wird das Tragen der erfindungsgemäßen Vorrichtung im Vergleich zu den Lösungen gemäß dem Stand der Technik komfortabler. Ferner kann das erfindungsgemäße Vorrichtung relativ kleinvolumig ausgebildet werden, da es unmittelbar hinter einem Ohr des Benutzers befestigt wird. Auch die Anbringung an dem Benutzerohr ist einfach durchführbar und kann mit einer Hand erfolgen.

Ebenso kann dadurch der optimale Druck- und Tragekomfort für den Träger individuell eingestellt werden. In einer Ausführungsform ist diese Verbindung eine Splintverbindung. Es kann jedoch auch als jede andere geeignete Verbindungsweise angewendet werden.

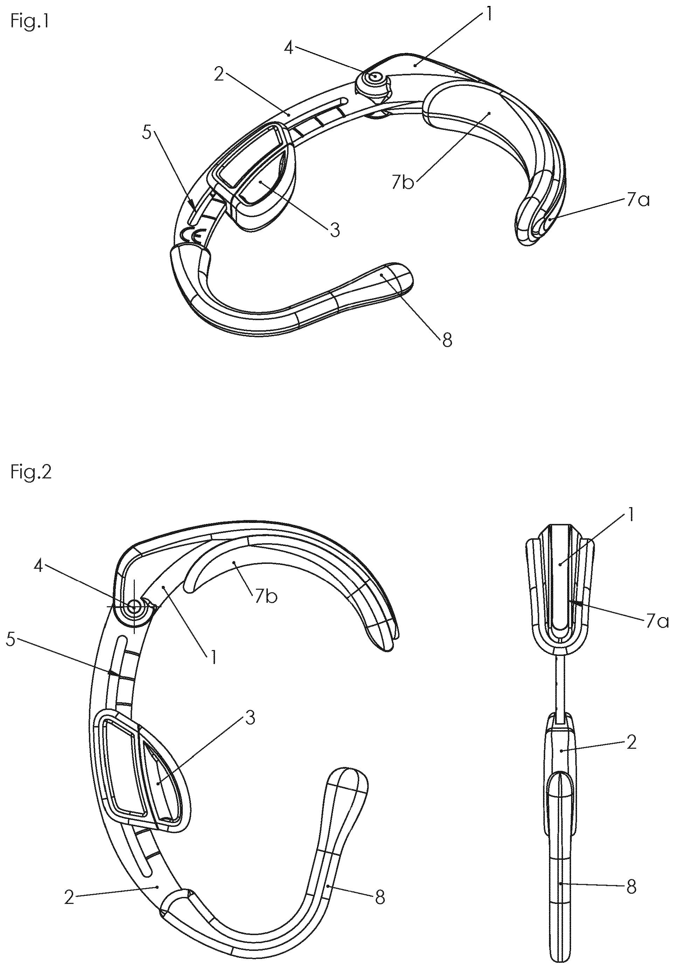

Eine Ausführungsform umfasst die Vorrichtung wie hierin beschrieben, wobei der untere Bügel einen Einschnitt 5 aufweist (Fig. 1). Dieser Einschnitt kann als Öffnung, Kerbe, Spalte, Schlitz, oder ähnlichem gestaltet sein. In diesem Einschnitt wird der Druckpunktkörper befestigt. Entlang des Einschnitts kann dann die Position des Druckpunktkörpers variabel verändert werden, sodass eine individuelle Anpassung des Druckpunktköpers an die Bedürfnisse des Trägers erfolgen kann.

Der Druckpunktkörper wird mit geeigneten Befestigungsmittel derart in diesem Einschnitt befestigt, sodass die Position entlang dieses Einschnitts variabel veränderbar ist. Beispielsweise kann der Druckpunktkörper einen Teil des Bügels komplett umschließen, oder beide Teile des Bügels umschließen. Der Druckpunktkörper kann beispielsweise mittels einer Klemmverbindung in der Spalte befestigt werden.

Da diese Vorrichtungen von den Patienten über einen längeren Zeitraum getragen werden sollen, ist ein angenehmer Tragekomfort sowie eine individuelle Anpassung der Position des Druckpunktkörpers unerlässlich. Zum einen, um den Heilungsfortschritt zu gewährleisten und zum anderen auch, um durch den erhöhten Tragekomfort für die notwendige Tragedauer über mehrere Wochen oder auch Monate durch den Patienten zu sorgen.

Eine Ausführungsform umfasst die Vorrichtung wie hierin beschrieben, wobei der obere Bügel eine Aussparung (Fig. 3) aufweist. In diese Aussparung können Inlays eingebracht werden. Durch diese Inlays wird ebenfalls der Tragekomfort für den Patienten verbessert.

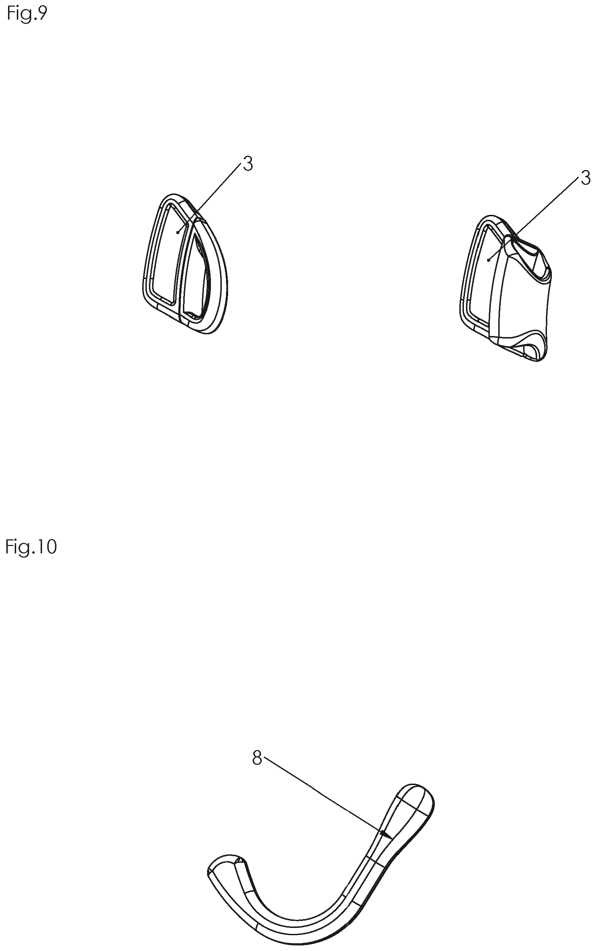

Eine Ausführungsform umfasst die Vorrichtung wie hierin beschrieben, wobei der untere Bügel 2 eine Ummantelung 8 aufweist (Fig. 10). Diese Ummantelung kann aus jedem beliebigen Material gefertigt werden, welches ein angenehmes Tragen der Vorrichtung unterstützt.

Eine Ausführungsform umfasst die Vorrichtung wie hierin beschrieben, wobei der untere Bügel 2 eine gewisse Flexibilität aufweist. Das heißt, der untere Bügel kann beispielsweise zusätzlich neben dem Druckpunktkörper gleichfalls einen Druck auf das Ohr ausüben. Dies kann beispielsweise dazu führen, dass der Effekt zur Linderung des Tinnitus-Leidens erhöht wird.

Die erfindungsgemäßen Vorrichtung ist zur Behandlung eines Tinnitus-Leidens geeignet.

Die erfindungsgemäßen Vorrichtung kann zur Behandlung eines Tinnitus-Leidens verwendet werden, wobei durch die Vorrichtung, insbesondere durch den Druckpunktkörper, die Stellung der Ohrmuschel (Außenohr) verändert wird.

Die Vorrichtung ist insbesondere geeignet zur Behandlung eines Tinnitus-Leidens, wobei durch die Vorrichtung, insbesondere durch den Druckpunktkörper, der Eintrittswinkel des Schalls am Außenohr verändert wird.

Mittels der erfindungsgemäßen Vorrichtung, insbesondere durch den Druckpunktkörper, wird Druck auf die Ohrmuschel (Außenohr) ausgeübt wird. Durch diesen Druck kann das Mittelohr deformiert werden und so der Schall anders gebrochen werden, wodurch störende Geräusche gemildert werden und letztendlich eine Linderung und in weiterer Folge sogar eine Heilung eines Tinnitus-Leidens ermöglich wird.

Stress bezeichnet zum einen durch spezifische äußere Reize (Stressoren) hervorgerufene psychische und physische Reaktionen bei Lebewesen, die zur Bewältigung besonderer Anforderungen befähigen und zum anderen die dadurch entstehende körperliche und geistige Belastung. Unter Stress versteht man die Beanspruchung und damit einhergehend die Auswirkung der Belastungen des Menschen durch innere und äußere Reize oder Belastungen. Diese können sowohl künstlich als auch natürlich, sowohl biotisch als auch abiotisch sein, sowohl auf den Körper als auch die Psyche des Menschen einwirken und letztlich als positiv oder negativ empfunden werden oder sich auswirken. Die Bewältigung der Beanspruchung ist von den persönlichen auch gesundheitlichen Eigenschaften und kognitiven Fähigkeiten der individuellen Person abhängig.

Stress wirkt sich auf die Psyche genauso aus wie auf die Befindlichkeit des Körpers. Es kann zu leichten und schweren Krankheiten kommen, wie beispielsweise zu Vergesslichkeit und Konzentrationsstörungen, Schlafstörungen, eingeschränkte Leistungsfähigkeit und Kreativität, nervösem unruhiges Verhalten, hastigem Sprechen und Essen, Ruhelosigkeit, Entscheidungsschwierigkeiten, Kommunikationsschwierigkeiten, Multitasking, Aktivismus, mangelnder Planung, Unordnung, Schwierigkeiten im Zeitmanagement, "nicht fertig werden", Arbeit mit nach Hause nehmen, Ineffizienz, Nicht-Abschalten-Können, Verzicht auf Urlaub, Reduktion der Freizeit, Vernachlässigung von Partnerschaft und Familie, Verzicht auf Bewegung, unkontrolliertem Essen und Rauchen, vermehrtem Alkoholkonsum, übermäßigem Kaffeegenuss, Einnahme von Schmerztabletten, Beruhigungsmitteln, Schlafmitteln und Aufputschmitteln, ständiger Sorge und "Zukunftsdenken", sich gehetzt und unter Druck fühlen, Gedankenkreisen und Grübeln, nächtlichem Denken an berufliche Inhalte, Denkblockade ("black out"), innerer Leere, Angst, Erschöpfung, innerer Unruhe und Anspannung, Reizbarkeit und Aggression, Stimmungsschwankungen, Unzufriedenheit und gedrückter Stimmung.

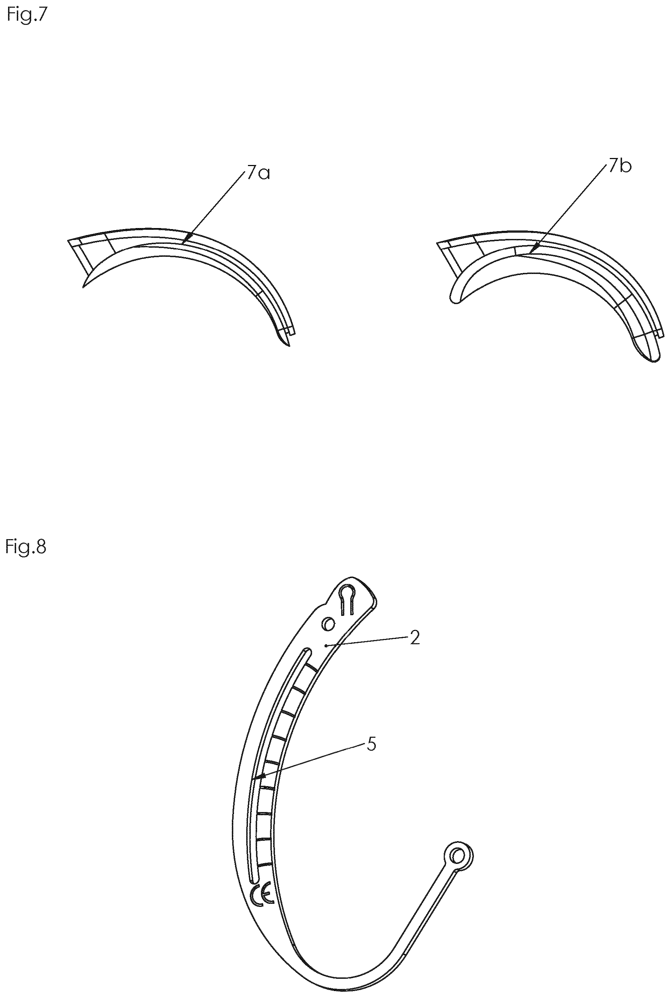

Das vegetative Nervensystem besteht aus zwei Komponenten welche gleichzeitig aktiv sind. Ein Teil, der sogenannte Sympathikus, sorgt für Anspannung, der andere Teil, der Parasympathikus für Entspannung sorgt. Stress führt zu Anspannung und bei dauerhafter Anspannung kippt das vegetative Nervensystem hierdurch in einen Modus der Überaktivierung des Sympathikus während der Parasympathikus für die Reduktion des Stresses im menschlichen Körper verantwortlich zeichnet. Dieser untere Bügelbereich (Fig. 2, 8) liegt am Ohr an einer Stelle an, an dem auch das vegetative Nervensystem, genauer gesagt der Parasympathikus mit stimuliert wird. Aufgrund dieser Stimulierung konnte bei den Trägern des erfindungsgemäßen Bügels eine Reduzierung des Stresses beobachtet werden.

### Kurze Beschreibung der Zeichnungen

Figur 1 zeigt die Vorrichtung in Form eines Bügels, bestehend aus einem unteren und oberen Bügel, und einem Druckpunktkörper.
Figur 2 zeigt eine Seiten- und Vorderansicht der Vorrichtung, bestehend aus einem unteren und oberen Bügel, und einem Druckpunktkörper.
Figur 3 zeigt eine Seiten- und Vorderansicht des oberen Bügels mit Inlays.
Figur 4 zeigt eine Seitenansicht der geöffneten Vorrichtung.

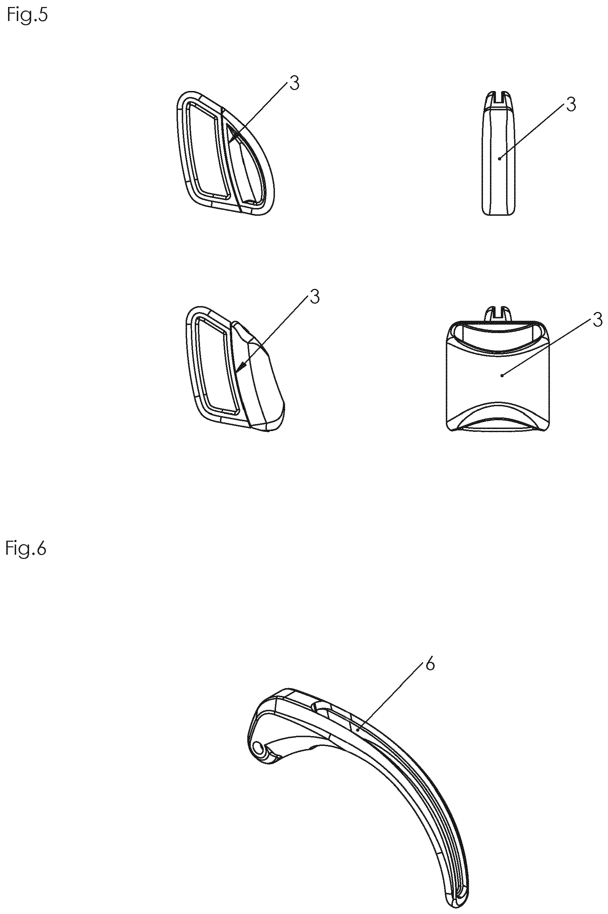

Figur 5 zeigt eine Seiten- und Vorderansicht von unterschiedlich gestalteten Druckpunktkörpern.
Figur 6 zeigt den oberen Bügel der Vorrichtung.
Figur 7 zeigt unterschiedliche Inlays.
Figur 8 zeigt den unteren Bügel mit Schlitz.
Figur 9 zeigt unterschiedlich gestaltete Druckpunktkörper.
Figur 10 zeigt eine Ummantelung für den unteren Teil des unteren Bügels.
Figur 11 zeigt die Trendanalyse des Stresslevels bei 36 Nutzern.

### Detaillierte Beschreibung der Erfindung

Die Auswirkungen eines Tinnitus richten sich stark nach der subjektiven Wahrnehmung und Beurteilung der Ohrgeräusche. Diese können einseitig oder beidseitig auftreten.

Tinnitus aurium bedeutet "das Klingeln der Ohren". Medizinisch definiert ist der Tinnitus als akustische Wahrnehmung, die ohne entsprechenden akustischen Reiz von außerhalb des Körpers entsteht und keinen Informationsgehalt besitzt.

Prinzipiell werden zwei Formen unterschieden. Beim objektiven Tinnitus gibt es eine körpereigene Schallquelle im Ohr oder in der Nähe des Ohrs, deren Schallaussendungen wahrgenommen werden. Das heißt, die oft von den Blutgefäßen oder der Muskulatur ausgehenden Geräusche existieren tatsächlich und sind somit auch für andere hörbar, wenn auch meist nur mit dem Stethoskop oder anderen medizinischen Geräten.

Sehr viel häufiger ist aber der subjektive Tinnitus. Hier nehmen die Betroffenen Töne und Geräusche wahr, die sich nicht auf eine physikalische Schallquelle zurückführen lassen und deshalb auch für andere Menschen nicht zu hören sind. Das bedeutet aber keineswegs, dass die Patienten sich das Brummen, Summen, Pfeifen, Klingeln, Rauschen oder Klopfen nur einbilden. Der subjektive Tinnitus ist vielmehr auf eine fehlerhafte Informationsbildung bzw. -verarbeitung im auditorischen System zurückzuführen, das sich vom Ohr über den Hörnerv bis zu den Hörzentren im Gehirn erstreckt.

Bei vielen Betroffenen lässt sich allerdings gar nicht definitiv feststellen, worauf die Ohrgeräusche zurückzuführen sind. Dies wird als idiopathischer Tinnitus bezeichnet.

Überraschenderweise wurde nun gefunden, dass durch Veränderung der Stellung des Außenohrs zum restlichen Ohr der Schall verändert wird, das heißt, der Schall wird im Vergleich zum "Normaleintritt" gebrochen. Dieses führt zu einem anderen Auftrittspunkt am Trommelfell. Dadurch wird der Hammer(griff) des ersten Gehörknochens anders bewegt, und sendet veränderte Drucksignale an die nächsten Gehörknochen bzw. dann weiter an die Gehörschnecke. In der Gehörschnecke werden die in einer Flüssigkeit gelagerten Sinneshärchen verändert in Bewegung versetzt. Dieses führt zu einer veränderten Umwandlung der elektrischen Signale ins Gehirn und zu einer lerntechnischen Veränderung der Synapsen - die bisherigen Töne werden nicht mehr gehört und somit langfristig "verlernt".

Durch Veränderungen des Schalleintrittswinkels werden die als "störend" wahrgenommenen Ohrgeräusche (Tinnitus) nicht mehr wahrgenommen, da der Schall auf anderen Auftrittspunkten am Trommelfell auftrifft.

Schall wird durch andere Brechung (Weglängenänderung) anders frequent. Frequenzänderung durch Abstandsänderung zwischen Beobachter (Trommelfell) und Schallquelle (Doppler Effekt). Der Schallweg im Ohr zwischen Außenohr und Trommelfell verändert sich. Daher werden die als störend empfundenen bisherigen "alten" Frequenzen nicht mehr wahrgenommen, da sich diese "frequent" verändert haben, und im Gehirn nicht mehr wahrgenommen werden. Dieses wird sich hauptsächlich bei hohen Frequenzen ergeben.

Mit der erfindungsgemäßen Vorrichtung wird der Schalleintrittswinkel so verändert, dass die Betroffenen die bisher gelernten störenden Töne nicht mehr hören. Die erfindungsgemäße Vorrichtung umfasst daher einen Bügel mit einem Druckpunktkörper, der so bemessen ist, dass er an der Ohrmuschel befestigt werden kann, wobei durch den Druckpunktkörper die Stellung der Ohrmuschel verändert wird. Durch die Änderung der Stellung des Außenohrs durch diesen Druckpunktkörper wird der Eintrittswinkel des Schalls in das Ohr verändert, wodurch die Patienten die bisherigen Töne werden nicht mehr höre und langfristig sogar verlernen, diese zu hören.

Insbesondere ist es vorteilhaft durch den Druckpunktkörper Druck auf das Außenohr auszuüben. Dieser Druck kann dazu führen, dass die Stellung des Außenohrs derart verändert wird, dass der Eintrittswinkel des Schalls ebenfalls geändert wird und so störende Tinnitus-Töne eben nicht mehr gehört werden. Die Wahrnehmung von Geräuschen, die Ihre Ursache nicht in akustischen Signalen aus der Umwelt haben, wird mit der erfindungsgemäßen Vorrichtung gemindert beziehungsweise gänzlich ausgeschaltet.

Der Druckpunktkörper wird individuell veränderbar auf der erfindungsgemäßen Vorrichtung angebracht, die an beziehungsweise hinter der Ohrmuschel befestigt werden kann. Der Druckpunktkörper kann unterschiedliche Formen und Größen aufweisen. Beispielhafte Druckpunktkörper sind in Figur 9 dargestellt.

Eine erfindungsgemäße Ausführungsform ist ein Bügel, bestehend aus einem oberen und einem unteren Bügelteil, die mittels einer Steckverbindung miteinander verbunden sind und geöffnet werden können, und der hinter dem Ohr getragen werden kann.

Der Druckpunktkörper kann aus unterschiedlichen Materialien hergestellt werden. Prinzipiell kann jedes Material verwendet werden, welches geeignet ist einen entsprechenden Formkörper zu bilden und angenehm zu tragen ist. Beispielsweise kann der Druckpunktkörper aus Kunststoff, Stoff, Filz, Schaumstoff, Gel, Gummi, elastischen Klebestreifen oder ähnlichen Materialien gefertigt sein. Der Druckpunktkörper kann aus einem aufgeschäumten Material; aus einem elastischen, deformierbaren Material, oder aus einem Plastikmaterial, beispielsweise aus Polypropylen hergestellt werden.

Der obere und der untere Bügel können aus stabilen Materialien gefertigt werden. Insbesondere geeignet sind beispielsweise Edelstahl, Titan, Federstahl, andere Metalle und metallische Legierungen, Kunststoffe, und dergleichen.

Um den Tragekomfort für die Patienten zu erhöhen, können beispielsweise in den oberen Bügel Inlays eingebracht werden (Figur 3). Diese Inlays bestehen aus einem geeigneten Material, wie beispielsweise aus Silikonen, Kautschuk, Kunststoff, Stoff, Filz, Schaumstoff, Gel, Gummi, oder ähnlichen Materialien.

Der untere Bügel kann teilweise ummantelt sein. Eine derartige Ummantelung (8) dient ebenfalls dem Tragekomfort. Die Ummantelung kann wiederum aus einem geeigneten Material gefertigt werden, insbesondere beispielsweise aus Gummi.

### Ausführungsbeispiele

Die nachfolgenden Ausführungsbeispiele illustrieren die vorliegende Erfindung ohne sie jedoch in ihrem Umfang zu beschränken

Eine Ausführungsform ist in Figur 1 dargestellt. Sie besteht aus einem oberen 1 und einem unteren Bügel 2, die mittels einer Steckverbindung 4 flexibel miteinander verbunden sind. Auf dem unteren Bügel 2 ist ein Druckpunktkörper 3 befestigt. Der Druckpunktkörper ist auf dem unteren Bügel beweglich montiert. Das heißt, der Druckpunktkörper kann entlang des oberen Teils des unteren Bügels 2 verschoben werden. Am oberen Bügel 1 sind optional Inlays angebracht. Der untere Bügel ist im unteren Teil ummantelt.

Figur 2 zeigt eine schematische Darstellung der Vorrichtung in geschlossener Form. Figur 4 zeigt eine schematische Darstellung der Vorrichtung in geöffneter Form, die das Anbringen der Vorrichtung hinter dem Ohr erleichtert.

In einer ersten Anwendungsbeobachtung mit 36 Nutzern, welche das den erfindungsgemäßen Bügel für mindestens 6 Wochen verwendet haben, wurde analysiert wie sich der Stresslevel der Nutzer verändert hat. Dabei haben die Nutzer ihren subjektiv empfundenen Stress auf einer Skala von 0 bis 10 täglich berichtet. Für jeden einzelnen Nutzer wurde eine lineare Trendanalyse gerechnet.

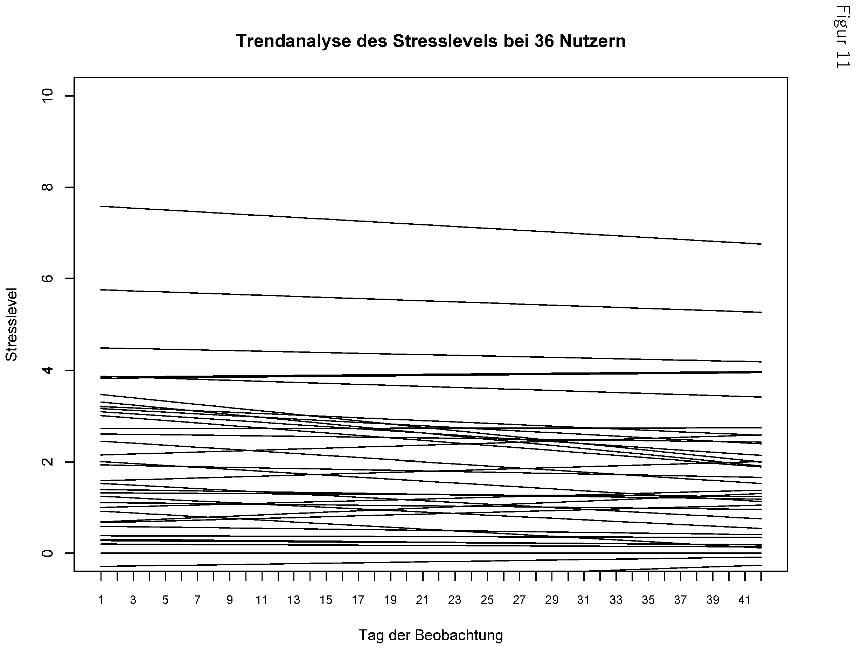

Bei 19 der 36 Nutzern (52,7%) hat sich, während der Nutzung des erfindungsgemäße Bügels der Stresslevel statistisch signifikant reduziert (siehe Figur 11).

## Patentansprüche

1. Vorrichtung zur Behandlung eines Tinnitus-Leidens und/oder Reduzierung von Stress bestehend aus einem oberen Bügel (1), einem unteren Bügel (2) und einem Druckpunktkörper (3), **dadurch gekennzeichnet, dass** der obere und untere Bügel mittels einer Steckverbindung (4) miteinander verbunden sind, wobei der untere Bügel einen horizontalen Einschnitt (5) aufweist in dem der Druckpunktkörper (3) befestigt ist, wobei der Druckpunktkörper (3) dazu ausgelegt ist, Druck auf das Außenohr auszuüben und die Stellung des Außenohrs zu verändern.

2. Vorrichtung nach Anspruch 1, wobei die Steckverbindung (4) eine Splintverbindung ist.

3. Vorrichtung nach Anspruch 1 oder 2, wobei der Druckpunktkörper (3) entlang des Einschnitts (5) frei beweglich ist.

4. Vorrichtung nach Anspruch 1, wobei der obere Bügel (1) eine Aussparung (6) aufweist.

5. Vorrichtung nach Anspruch 4, wobei in der Aussparung (6) ein oder zwei Inlays (7a, 7b) angebracht sind.

6. Vorrichtung nach Anspruch 1, wobei der untere Bügel (2) eine Ummantelung (8) aufweist.

7. Vorrichtung nach Anspruch 6, wobei die Ummantelung (8) aus einem weichen Material, bevorzugt aus Gummi besteht.

8. Vorrichtung nach Anspruch 1, wobei der untere Bügel (2) beweglich ist.

## Claims

1. A device for treating a tinnitus affliction and/or reducing stress, consisting of an upper hook (1), a lower hook (2) and a pressure point body (3), **characterised in that** the upper and lower hooks are connected to one another by means of a plug connection (4), wherein the lower hook has a horizontal recess (5) in which the pressure point body (3) is fastened, wherein the pressure point body (3) is designed to exert pressure on the outer ear and to change the position of the outer ear.

2. The device according to claim 1, wherein the plug connection (4) is a cotter pin connection.

3. The device according to claim 1 or 2, wherein the pressure point body (3) is freely movable along the recess (5).

4. The device according to claim 1, wherein the upper hook (1) has a cut-out (6).

5. The device according to claim 4, wherein one or two inlays (7a, 7b) are attached in the cut-out (6).

6. The device according to claim 1, wherein the lower hook (2) has a sheath (8).

7. The device according to claim 6, wherein the sheath (8) consists of a soft material, preferably rubber.

8. The device according to claim 1, wherein the lower hook (2) is movable.

## Revendications

1. Dispositif destiné au traitement de troubles acouphéniques et/ou à la réduction de stress, se composant d'une branche supérieure (1), d'une branche inférieure (2) et d'un corps de pression ponctuelle (3), **caractérisé en ce que** les branches supérieure et inférieure sont reliées entre elles par un assemblage à emboîtement (4), la branche inférieure présentant une découpe (5) horizontale dans laquelle est fixé le corps de pression ponctuelle (3), le corps de pression ponctuelle (3) étant conçu pour exercer une pression sur l'oreille externe et modifier ainsi la position de ladite oreille externe.

2. Dispositif selon la revendication 1, dans lequel l'assemblage à emboîtement (4) est un assemblage par goupille.

3. Dispositif selon la revendication 1 ou 2, dans lequel le corps de pression ponctuelle (3) peut être déplacé librement le long de la découpe (5).

4. Dispositif selon la revendication 1, dans lequel la branche supérieure (1) présente un évidement (6).

5. Dispositif selon la revendication 4, dans lequel un ou deux inserts (7a, 7b) sont mis en place dans l'évidement (6).

6. Dispositif selon la revendication 1, dans lequel la branche inférieure (2) présente une gaine (8).

7. Dispositif selon la revendication 6, dans lequel la gaine (8) est réalisée en un matériau souple, de préférence en caoutchouc.

8. Dispositif selon la revendication 1, dans lequel la branche inférieure (2) est mobile.
